# EUROPEAN PATENT APPLICATION

(11) **EP 4 721 790 A1**
(43) Date of publication of application: **08.04.2026**
(21) Application number: 23939455.4
(22) Date of filing: 26.05.2023
(51) Int. Cl.: A61M 15/00, A61M 13/00

(54) **POWDER INHALATION SYSTEM**

(71) Applicant: Japan Tobacco Inc., Tokyo 105-6927 (JP)
(72) Inventor: NAKANO, Takuma, Tokyo 130-8603 (JP); SADAKARI, Kei, Tokyo 130-8603 (JP); TAKANABE, Yurina, Tokyo 130-8603 (JP); FUJITA, Ryoji, Tokyo 130-8603 (JP); HODONO, Hiroshi, Tokyo 130-8603 (JP); WAKAMATSU, Miki, Tokyo 130-8603 (JP)
(74) Representative: Hoffmann Eitle
(86) International application number: PCT/JP2023/019627
(87) International publication number: WO 2024/246970

(57) **Abstract**

Provided is a powder inhalation system including a storage unit for storing a powder for inhalation, and a powder inhaler for inhaling the powder. The powder inhaler has a chamber in which the storage unit is disposed, and the storage unit stores a desiccant different from the powder.

## Description

### TECHNICAL FIELD

The present invention relates to a powder inhalation system.

### BACKGROUND ART

There are known inhalers for inhaling a powder containing nicotine, etc. (e.g., see PTL 1). In the inhaler disclosed in PTL 1, an opening is formed by a needle-shaped member in a capsule accommodating a powder so that a user can be supplied with the powder inside the capsule.

### CITATION LIST

### PATENT LITERATURE

PTL 1: WO 2022/195480 A1

### SUMMARY OF INVENTION

### TECHNICAL PROBLEM

However, it is conceivable that a powder accommodated in a capsule, as with inhalers such as in PTL 1, might absorb water and solidify, which is an unintended situation. If the powder inside the capsule solidifies, there is a risk that clumps of powder will not emerge from the opening formed in the capsule, or that even if clumps of powder do emerge, these may impart a sense of discomfort to the user when inhaled by the user.

In light of this, one objective of the present invention lies in inhibiting powder inside a capsule from absorbing water and solidifying.

### SOLUTION TO PROBLEM

A first aspect provides a powder inhalation system comprising an accommodating portion accommodating a powder to be inhaled; and a powder inhaler for inhaling the powder. The powder inhaler has a chamber in which the accommodating portion is disposed. The accommodating portion has a weakened portion with a lower breaking strength than other parts of the accommodating portion.

According to the first aspect, the accommodating portion comprises the weakened portion which has a relatively low breaking strength, and it is therefore possible to inhibit variations in the position or size of the opening because the weakened portion is ruptured when an opening is formed in the accommodating portion by a piercing element such as a needle.

The powder inhalation system may have a piercing element configured to form an opening in the accommodating portion disposed in the chamber, and the piercing element may be configured to form the opening in the weakened portion.

In this case, the opening can be easily formed in the weakened portion of the accommodating portion disposed in the chamber by means of the piercing element.

The shape of the weakened portion may be substantially consistent with a cross-sectional shape of the piercing element orthogonal to a longitudinal direction, or larger than this cross-sectional shape.

In this case, the opening may be formed by the piercing element only in the weakened portion. In other words, it is possible to inhibit an opening from being formed in a part of the accommodating portion other than the weakened portion.

The accommodating portion may have an elliptical or rounded rectangular external shape, as seen from a direction orthogonal to the longitudinal direction thereof, and the weakened portion may be provided on a major axis of the elliptical or rounded rectangular shape.

In this case, the weakened portion is positioned on an end face (tip) part of the accommodating portion, so the opening can be easily formed in the end face part of the accommodating portion. As a result, the powder accommodated in the accommodating portion can be easily discharged from the opening, and it is therefore possible to inhibit the powder from remaining inside the accommodating portion.

In a cross section orthogonal to a direction of insertion of the accommodating portion into the chamber, an outer diameter of the piercing element in said cross section may be larger than a difference between an inner diameter of the chamber and an outer diameter of the accommodating portion.

In this case, the piercing element can form an opening in the weakened portion even if the position of the accommodating portion is offset inside the chamber in a direction orthogonal to the insertion direction.

The powder inhaler may comprise: a sensor for detecting a pressure applied to the piercing element; and a control unit for determining whether or not the piercing portion has formed an opening in the weakened portion, based on the pressure detected by the sensor.

In this case, the control unit can determine whether or not an opening could be formed in the weakened portion of the accommodating portion, so if an opening has not been formed in the weakened portion, a notification to that effect can be provided to a user by emission of vibration, sound, or light, etc. from the powder inhaler, for example. The user can take appropriate measures such as replacing the accommodating portion if an opening has not been formed in the weakened portion.

The control unit may determine that the piercing element has formed an opening in the weakened portion when the pressure detected by the sensor is equal to or less than a predetermined value.

In this case, the user can be notified that the piercing element has formed an opening in the weakened portion by emission of vibration, sound, or light, etc. from the powder inhaler, for example. The user can start inhaling having confirmed that an opening has been formed in the weakened portion.

The weakened portion may be formed by a different material from that of other parts of the accommodating portion.

In this case, it is possible to make the breaking strength of the weakened portion lower than that of other parts due to the different materials.

The thickness of the weakened portion may be smaller than the thickness of other parts of the accommodating portion.

In this case, it is possible to make the breaking strength of the weakened portion lower than that of other parts due to the different thicknesses.

The accommodating portion may include a first part and a second part configured to be detachable from the first part, and the weakened portion may be provided on either one of the first part and the second part.

In this case, the accommodating portion accommodating the powder can be easily manufactured.

The entirety of either one of the first part and the second part may constitute the weakened portion.

In this case, the first part or the second part can be easily manufactured because an entire part of the accommodating portion can be formed as the weakened portion, rather than just part of the material constituting the accommodating portion.

The accommodating portion may be detachably received in the chamber of the powder inhaler.

In this case, the used accommodating portion can be removed from the chamber and discarded or replaced.

The accommodating portion may be integrally formed with the powder inhaler.

In this case, the accommodating portion can be discarded or replaced together with the powder inhaler, after the powder has been inhaled.

The powder inhalation system may comprise a mouthpiece having an inhalation port; and a transport line for transporting the powder in the accommodating portion toward the inhalation port.

In this case, the user inhales from the mouthpiece, and the powder in the accommodating portion can thereby be supplied into the user's mouth through the transport line.

The powder inhalation system may comprise a cartridge which holds the accommodating portion and is detachable from the powder inhaler, and a portion of the cartridge may extend to outside of the powder inhaler in a state in which the accommodating portion is disposed in the chamber.

In this case, the used accommodating portion can be discarded or replaced together with the cartridge comprising the accommodating portion. Furthermore, the user can remove the cartridge from the powder inhaler by gripping the part of the cartridge extending to outside of the powder inhaler, enabling the user to easily discard or replace the cartridge and the accommodating portion.

A second aspect provides a powder inhalation system comprising an accommodating portion accommodating a powder to be inhaled; and a powder inhaler for inhaling the powder. The powder inhaler has a chamber in which the accommodating portion is disposed, and the accommodating portion accommodates a desiccant different from the powder.

The second aspect makes it possible to inhibit the powder inside the accommodating portion from absorbing moisture and solidifying.

The desiccant may have higher moisture absorption than the powder.

In this case, the desiccant can efficiently inhibit the powder from absorbing moisture. It should be noted that the moisture absorption may be measured by means of a drying loss method, for example.

A piercing element configured to form an opening in the accommodating portion disposed in the chamber may be provided.

In this case, the opening can be easily formed in the accommodating portion disposed in the chamber by means of the piercing element.

The particle size of the desiccant may be larger than the opening formed in the accommodating portion.

In this case, the desiccant is inhibited from being discharged from the opening formed by the piercing element, so the user can be inhibited from inhaling the desiccant by mistake.

The hardness of the desiccant may be higher than the hardness of the powder.

In this case, the desiccant collides with the powder inside the accommodating portion, thereby making it possible to loosen solidified powder. Furthermore, the desiccant itself is inhibited from being broken up and refined, so it is possible to inhibit the refined desiccant from passing through the opening and being supplied to the user.

The desiccant may be packaged in an air-permeable member and accommodated in the accommodating portion.

In this case, the desiccant is packaged, so it is possible to prevent the desiccant from mixing with the powder and to prevent the user from inhaling the desiccant. Moreover, a nonwoven fabric or paper, such as plain paper, may be used as the air-permeable member, for example.

The desiccant may comprise at least one selected from the group consisting of: silica gel, sepiolite, calcium oxide, diatomaceous earth, activated charcoal, activated clay, zeolite, white carbon, calcium chloride, magnesium chloride, potassium acetate, disodium phosphate, sodium citrate, and water-absorbent polymers.

In this case, a highly water-absorbent material is used as the desiccant, so it is possible to further inhibit the powder inside the accommodating portion from absorbing water and solidifying.

The accommodating portion may be detachably received in the chamber of the powder inhaler.

In this case, the used accommodating portion can be removed from the chamber and discarded or replaced.

The accommodating portion may be integrally formed with the powder inhaler.

In this case, the accommodating portion can be discarded or replaced together with the powder inhaler, after the powder has been inhaled.

The powder inhalation system may comprise a mouthpiece having an inhalation port; and a transport line for transporting the powder in the accommodating portion toward the inhalation port.

In this case, the user inhales from the mouthpiece, and the powder in the accommodating portion can thereby be supplied into the user's mouth through the transport line.

The powder inhalation system may comprise a cartridge which holds the accommodating portion and is detachable from the powder inhaler, and a portion of the cartridge may extend to outside of the powder inhaler in a state in which the accommodating portion is disposed in the chamber.

In this case, the used accommodating portion can be discarded or replaced together with the cartridge comprising the accommodating portion. Furthermore, the user can remove the cartridge from the powder inhaler by gripping the part of the cartridge extending to outside of the powder inhaler, enabling the user to easily discard or replace the cartridge and the accommodating portion.

A third aspect provides a powder inhalation system comprising: an accommodating portion accommodating a powder to be inhaled; a mouthpiece having an inhalation port; a transport line for transporting the powder inside the accommodating portion from the accommodating portion to the inhalation port; a wall portion defining the transport line; and a powder inhaler for inhaling the powder. The powder inhaler comprises a chamber in which the accommodating portion is disposed, and a piercing element configured to form an opening in the accommodating portion disposed in the chamber. The wall portion defining the transport line is formed by a transparent or semitransparent material which makes the inside of the transport line visible from the outside.

According to the third aspect, the powder passing through the transport line is visible from the outside, so it is possible to easily confirm whether or not there is powder remaining inside the accommodating portion or the chamber. It is therefore possible to easily judge whether or not the powder inhalation system is spent.

The powder inhalation system may comprise a cartridge for holding the accommodating portion, at least a portion of the cartridge extending to outside of the powder inhaler, and the cartridge may be detachable from the powder inhaler and may comprise the mouthpiece, the transport line, and the wall portion.

In this case, the powder passing through the transport line in the cartridge is visible from the outside, so it is possible to easily confirm whether or not there is powder remaining inside the accommodating portion or the chamber. It is therefore possible to easily judge whether or not the powder inhalation system is spent. In addition, the used accommodating portion can be discarded or replaced together with the cartridge comprising the accommodating portion. Furthermore, the user can remove the cartridge from the powder inhaler by gripping the part of the cartridge extending to outside of the powder inhaler, enabling the user to easily discard or replace the cartridge and the accommodating portion.

The wall portion of the cartridge may be formed by at least one selected from the group consisting of glassine paper, parchment paper, paraffin paper, cellophane, polypropylene film, polyvinyl chloride film, and cellulose acetate film.

In this case, the wall portion of the cartridge can be formed easily and inexpensively.

The wall portion of the cartridge may be formed by an antistatic material or coated with an antistatic material.

In this case, the wall portion of the cartridge is charged and can thereby inhibit the powder passing through the transport line from adhering to the wall portion. This allows the powder to be supplied to the user without waste. An anionic antistatic agent, cationic antistatic agent, amphoteric antistatic agent, or nonionic antistatic agent, etc. may be used as the antistatic material, for example.

The accommodating portion may be integrally formed with the powder inhaler.

In this case, the accommodating portion can be discarded or replaced together with the powder inhaler, after the powder has been inhaled.

The accommodating portion may be detachably received in the chamber of the powder inhaler.

In this case, the used accommodating portion can be removed from the chamber and discarded or replaced.

The powder inhaler may comprise the transport line and the wall portion.

In this case, the powder passing through the transport line in the powder inhaler is visible from the outside, so it is possible to easily confirm whether or not there is powder remaining inside the accommodating portion or the chamber. It is therefore possible to easily judge whether or not the powder inhalation system is spent.

The entirety of the wall portion of the powder inhaler may be formed by the transparent or semitransparent material.

In this case, the powder passing through the transport line is visible from the outside over the entirety of the transport line, so it is possible to confirm even more easily whether or not there is powder remaining inside the accommodating portion or the chamber.

The wall portion of the powder inhaler may be formed by at least one selected from the group consisting of an acrylic resin, polycarbonate, polyethylene terephthalate, polyvinyl chloride, and polystyrene.

In this case, the wall portion of the powder inhaler can be formed easily and inexpensively.

The accommodating portion may be formed by a transparent or semitransparent material which makes the inside of the accommodating portion visible from the outside.

In this case, the inside of the accommodating portion is visible from the outside, so it is possible to easily confirm whether or not there is powder remaining in the accommodating portion.

The part of the accommodating portion in which the opening is formed may be formed by the transparent or semitransparent material.

In this case, it is possible to visually check from the outside whether or not the powder is being discharged from the opening in the accommodating portion, so it is possible to confirm even more easily whether or not there is powder remaining in the accommodating portion.

A fourth aspect provides a powder inhalation system comprising an accommodating portion accommodating a powder to be inhaled; and a powder inhaler for inhaling the powder. This powder inhalation system comprises a mouthpiece having an inhalation port, and a transport line for transporting the powder in the accommodating portion toward the inhalation port; the powder inhaler comprises a chamber in which the accommodating portion is disposed, and a piercing element configured to form an opening in the accommodating portion disposed in the chamber; and the flavor inhaler system has an obstructing portion which is disposed in the transport line and obstructs part of a flow of powder when the user inhales.

According to the fourth aspect, even if the powder inside the accommodating portion absorbs water to form clumps, the obstructing portion can inhibit such clumps from reaching the user's mouth.

The obstructing portion may comprise a columnar body having a plurality of grooves on an outer circumferential surface thereof, the plurality of grooves may extend toward the inhalation port, and at least one of the plurality of grooves may have a dam portion provided within said groove.

The obstructing portion may comprise a mesh filter.

In this case, the powder passes through the mesh filter and reaches the inhalation port, but clumps of powder can be inhibited from reaching the inhalation port by the mesh filter. This makes it possible to inhibit clumps of powder from reaching the user's mouth.

The obstructing portion may comprise a collision plate which is provided on an inner face of the wall portion defining the transport line, and forms an opening in the center of the transport line.

In this case, the powder passes through the open central part of the transport line to reach the inhalation port, but clumps of powder can be inhibited from reaching the inhalation port by the collision plate. This makes it possible to inhibit clumps of powder from reaching the user's mouth.

The collision plate may be curved or inclined toward the accommodating portion.

In this case, the curved or inclined collision plate can efficiently inhibit clumps of powder from reaching the inhalation port.

The obstructing portion may be formed by an antistatic material or coated with an antistatic material.

In this case, the obstructing portion is charged and can thereby inhibit the powder passing through the transport line from adhering to the obstructing portion. This allows the powder to be supplied to the user without waste. An anionic antistatic agent, cationic antistatic agent, amphoteric antistatic agent, or nonionic antistatic agent, etc. may be used as the antistatic material, for example.

The obstructing portion may be formed by a non-water-absorbent material or coated with a non-water-absorbent material.

In this case, it is possible to inhibit the powder solidifying as a result of the powder absorbing water when the powder collides with the obstructing portion. Furthermore, it is possible to inhibit the powder from adhering to the obstructing portion when the powder collides with the obstructing portion. This makes it possible to supply the powder to the user without waste, while inhibiting clumps of powder from reaching the user's mouth. It should be noted that polyethylene or polypropylene, etc. may be used as the non-water-absorbent material, for example.

The accommodating portion may be detachably received in the chamber of the powder inhaler.

In this case, the used accommodating portion can be removed from the chamber and discarded or replaced.

The accommodating portion may be integrally formed with the powder inhaler.

In this case, the accommodating portion can be discarded or replaced together with the powder inhaler, after the powder has been inhaled.

A cartridge which holds the accommodating portion and comprises the transport line may be provided, and the obstructing portion may be provided in the cartridge.

In this case, the used accommodating portion can be discarded or replaced together with the cartridge comprising the accommodating portion. Furthermore, the obstructing portion is provided in the cartridge, which is replaced as appropriate, so there is no need to clean the obstructing portion even if powder adheres thereto.

A portion of the cartridge may extend to outside of the powder inhaler in a state in which the accommodating portion is disposed in the chamber.

In this case, the user can remove the cartridge from the powder inhaler by gripping the part of the cartridge extending to outside of the powder inhaler, enabling the user to easily discard or replace the cartridge and the accommodating portion.

The powder inhaler may comprise the transport line, and the obstructing portion may be provided in the powder inhaler.

In this case, there is no need to provide an obstructing portion in the cartridge, so the obstructing portion can be used repeatedly.

### BRIEF DESCRIPTION OF DRAWINGS

Fig. 1 is a schematic view in side section showing a powder inhalation system according to a first embodiment.
Fig. 2 is a schematic view in side section showing the powder inhalation system according to another example of the first embodiment.
Fig. 3 is a schematic diagram showing an accommodating portion.
Fig. 4 is a schematic view in side section of an accommodating portion according to a second embodiment.
Fig. 5 is a schematic view in side section of the accommodating portion according to another example of the second embodiment.
Fig. 6 is a schematic view in side section showing a powder inhalation system according to a third embodiment.
Fig. 7 is a schematic view in side section showing the powder inhalation system according to another example of the third embodiment.
Fig. 8 is a schematic side view showing an example of an accommodating portion in the third embodiment.
Fig. 9 is a schematic view in side section showing a powder inhalation system according to a fourth embodiment.
Fig. 10 is an oblique view of a filter.
Fig. 11 is a schematic view in side section showing another example of the powder inhalation system according to the fourth embodiment.
Fig. 12 is a schematic view in side section showing another example of the powder inhalation system according to the fourth embodiment.
Fig. 13 is a schematic view in side section of an example of a cartridge according to the fourth embodiment.
Fig. 14 is a schematic view in side section of an example of a cartridge according to the fourth embodiment.
Fig. 15 is a schematic view in side section of an example of a cartridge according to the fourth embodiment.

### DESCRIPTION OF EMBODIMENTS

Embodiments of the present invention will be described below with reference to the drawings. In the drawings described below, identical or corresponding components are assigned the same reference numbers and duplicate descriptions will not be given. In the specification of this case, "longitudinal direction" refers to a direction of insertion when an accommodating portion 10 is accommodated in a chamber 102, a direction of extension of a piercing member 132, or a direction of extension of a tubular member 22 of a cartridge 20, which will be described later.

### First Embodiment

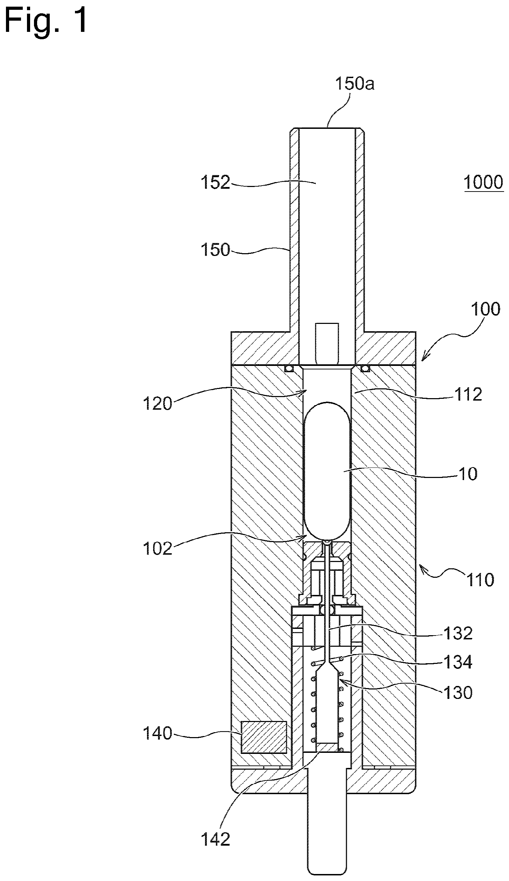

Fig. 1 is a schematic view in side section showing a powder inhalation system 1000 according to a first embodiment. As shown in fig. 1, the powder inhalation system 1000 comprises: an accommodating portion 10 containing a powder to be inhaled; and a powder inhaler 100 for inhaling the powder. The powder inhaler 100 has a chamber 102 in which the accommodating portion 10 is disposed. Furthermore, the powder inhaler 100 comprises an enclosure 110 defining the chamber 102, for example. The accommodating portion 10 may be detachably received in the chamber 102. In this case, the used accommodating portion 10 can be removed from the chamber 102 and discarded or replaced. Alternatively, the accommodating portion 10 may be integrally formed with the powder inhaler 100. In this case, the accommodating portion 10 can be discarded or replaced together with the powder inhaler 100, after the powder has been inhaled.

There is no particular limitation as to the powder, provided that it is a substance which can be inhaled by the user. The powder may be a flavor powder containing a flavor, or a medication such as a drug. When the powder is a flavor powder, there is no particular limitation as to the type thereof provided that it contains a flavor and can be inhaled. The powder may comprise nicotine, various types of flavoring materials such as menthol, sugars, or amino acids. The powder may be produced by means of spray drying. Sugar alcohols and amino acids may be used as excipients of the powder. Specifically, excipients may be selected from mannitol, trehalose, leucine, alanine, valine, isoleucine, methionine, phenylalanine, tyrosine, and tryptophan, which may also be used in combination. When the powder comprises a flavor powder, the powder inhaler 100 is constructed as a flavor inhaler for inhaling the flavor, and the accommodating portion 10 is constructed as a flavor-generating article. As a result, the powder inhaler 100 can provide the user with the opportunity to enjoy the flavor.

Furthermore, the powder inhalation system 1000 preferably comprises a mouthpiece 150 having an inhalation port 150a; and a transport line 120 for transporting the powder in the accommodating portion 10 toward the inhalation port 150a. In this case, the user inhales from the mouthpiece 150, and the powder in the accommodating portion 10 can thereby be supplied into the user's mouth through the transport line 120. In the example depicted, the powder inhaler 100 comprises the mouthpiece 150 and the transport line 120. The enclosure 110 of the powder inhaler 100 comprises a wall portion 112 defining the transport line 120. The mouthpiece 150 comprises a passage 152 communicating with the transport line 120 inside the enclosure 110. The passage 152 forms part of the transport line 120. The mouthpiece 150 may be configured to be detachable from the enclosure 110.

The powder inhalation system 1000 preferably comprises a piercing element 130 configured to form an opening in the accommodating portion 10 disposed in the chamber 102. In this embodiment, the piercing element 130 is provided in the powder inhaler 100, but this is not limiting, and the user may equally use a piercing element 130 which is not provided in the powder inhaler 100 in order to form the opening in the accommodating portion 10. When the opening is formed in the accommodating portion 10, the powder is discharged into the chamber 102 from the opening in the accommodating portion 10, and the user inhales from the mouthpiece 150, whereby the powder is supplied into the user's mouth through the transport line 120. Note that the powder discharged into the chamber 102 is able to move to the mouthpiece 150 by passing between the outer circumferential surface of the accommodating portion 10 and the wall portion 112 defining the transport line 120.

In this embodiment, the piercing element 130 may comprise: a piercing member 132 which extends in a first direction (longitudinal direction) in which the accommodating portion 10 and the piercing element 130 are adjacent; and a biasing member 134 which biases the piercing member 132 in a direction away from the accommodating portion 10 in the first direction. The piercing member 132 may be any member capable of forming an opening in the accommodating portion 10, such as a needle or a pin, for example. When the opening is formed in the accommodating portion 10, the user pushes the piercing member 132 in order to move it toward the accommodating portion 10 disposed in the chamber 102. This ruptures part of the accommodating portion 10 so that the opening is formed. The piercing member 132 which has been moved toward the accommodating portion 10 is biased by the biasing member 134 and returns to its original position.

As shown in fig. 1, the powder inhaler 100 preferably comprises: a sensor 142 for detecting a pressure applied to the piercing element 130; and a control unit 140 for determining whether or not the piercing element 130 has formed an opening in the accommodating portion 10, based on the pressure detected by the sensor 142. In this case, the control unit 140 can determine whether or not an opening could be formed in the accommodating portion 10, so if an opening could not be formed in the accommodating portion 10, a notification to that effect can be provided to the user by emission of vibration, sound, or light, etc. from the powder inhaler, for example. On receiving this notification, the user can make the decision to start inhaling.

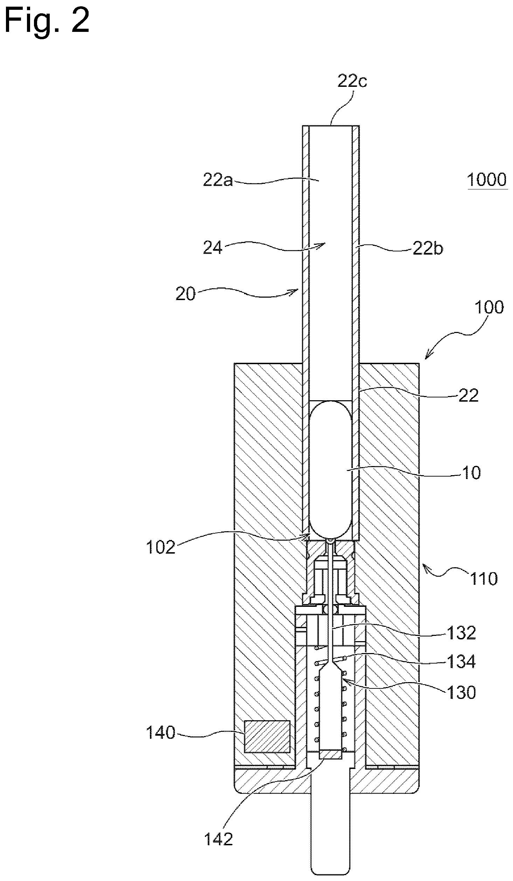

Fig. 2 is a schematic view in side section showing the powder inhalation system 1000 according to another example of the first embodiment. The example shown in fig. 2 differs from the example shown in fig. 1 in that, in the example shown in fig. 2, the accommodating portion 10 is held by means of a cartridge 20, and the powder inhaler 100 does not comprise the mouthpiece 150. Specifically, in the example shown in fig. 2, the cartridge 20 which holds the accommodating portion 10 is detachable from the powder inhaler 100, and a portion of the cartridge 20 extends to outside of the powder inhaler 100 in a state in which the accommodating portion 10 is disposed in the chamber 102, as shown in fig. 2. In this case, the used accommodating portion 10 can be discarded or replaced together with the cartridge 20 comprising the accommodating portion 10. Furthermore, the user can remove the cartridge 20 from the powder inhaler 100 by gripping the part of the cartridge 20 extending to outside of the powder inhaler 100, enabling the user to easily discard or replace the cartridge 20 and the accommodating portion 10.

Specifically, the cartridge 20 comprises a tubular member 22 which internally holds the accommodating portion 10. The tubular member 22 may be formed by paper or a resin, etc., for example. As shown in the drawing, the accommodating portion 10 is preferably held in the vicinity of one end portion of the tubular member 22. This allows the accommodating portion 10 to be positioned so that the piercing member 132 can form an opening in the accommodating portion 10 when the cartridge 20 has been inserted into the chamber 102 of the powder inhaler 100. Furthermore, in the example depicted, the cartridge 20 comprises a mouthpiece 22a having an inhalation port 22c; a transport line 24 for transporting the powder in the accommodating portion 10 toward the inhalation port 22c; and a wall portion 22b. Specifically, a portion of the tubular member 22 at the end on the opposite side to the end which is inserted into the chamber 102 constitutes the mouthpiece 22a. Furthermore, the powder which is discharged after the opening has been formed in the accommodating portion 10 passes through the transport line 24 inside the tubular member 22 and reaches the user's mouth. In the example depicted, the tubular member 22 therefore functions as the wall portion 22b defining the transport line 24.

The wall portion 22b is preferably formed by an antistatic material or coated with an antistatic material. In this case, the wall portion 22b of the cartridge 20 is charged and can thereby inhibit the powder passing through the transport line 24 from adhering to the wall portion 22b. This allows the powder to be supplied to the user without waste. An anionic antistatic agent, cationic antistatic agent, amphoteric antistatic agent, or nonionic antistatic agent, etc. may be used as the antistatic material, for example.

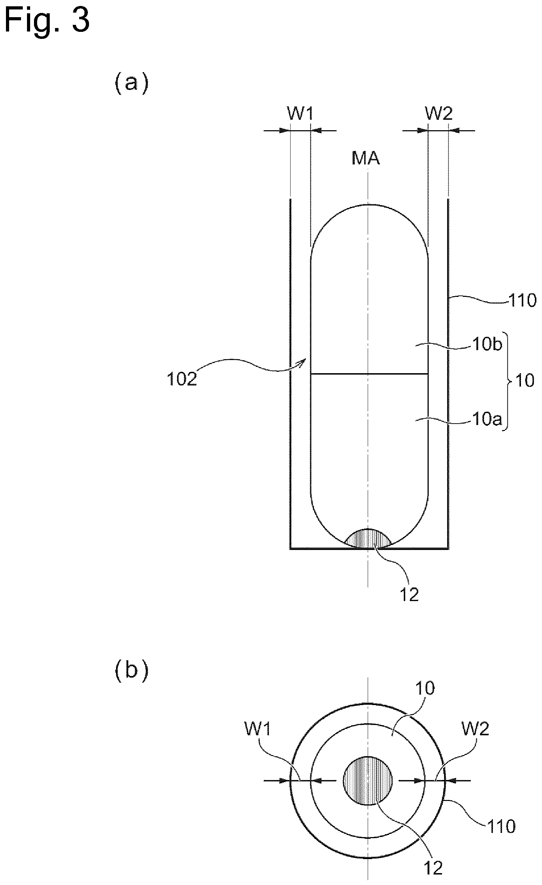

Fig. 3 is a schematic diagram showing the accommodating portion 10. Fig. 3(a) is a schematic view in side section of the accommodating portion 10 while it is accommodated in the chamber 102. Fig. 3(b) is an end face view of the accommodating portion 10. The accommodating portion 10 may be a capsule-shaped container configured to accommodate the powder, for example. This is not limiting, and the accommodating portion 10 may be a container of any shape, for example a spherical, box-like, or bag-like container. In this embodiment, the accommodating portion 10 has a weakened portion 12 with a lower breaking strength than other parts of the accommodating portion 10, in order to inhibit variations in the position or size of the opening, when an opening is formed in the accommodating portion 10. As a result, it is possible to inhibit variations in the position or size of the opening because the weakened portion 12 is ruptured when an opening is formed in the accommodating portion 10 by the piercing element 130 such as a needle. Note that a greater pressure being exerted on the piercing element 130 when an opening is formed by the piercing element 130 means a higher "breaking strength", and a smaller pressure being exerted on the piercing element 130 when an opening is formed by the piercing element 130 means a lower "breaking strength". The piercing element 130 shown in fig. 1 and 2 is preferably configured to form the opening in the weakened portion 12. In this case, the opening can be easily formed in the weakened portion 12 of the accommodating portion 10 disposed in the chamber 102 by means of the piercing element 130.

The accommodating portion 10 may be formed by paper or a synthetic resin, etc., for example. The weakened portion 12 may be formed by a different material from that of other parts of the accommodating portion 10. In this case, it is possible to make the breaking strength of the weakened portion 12 lower than that of other parts due to the different materials. Furthermore, the thickness of the weakened portion 12 may be smaller than the thickness of other parts of the accommodating portion 10. In this case, it is possible to make the breaking strength of the weakened portion 12 lower than that of other parts due to the different thicknesses.

Furthermore, as shown in fig. 3, the accommodating portion 10 preferably includes a first part 10a and a second part 10b configured to be detachable from the first part 10a. In this case, the accommodating portion 10 accommodating the powder can be easily manufactured. The weakened portion 12 may be provided on either one of the first part 10a and the second part 10b. In the example shown in fig. 3, the weakened portion 12 is provided on the first part 10a. Alternatively, the entirety of either one of the first part 10a and the second part 10b may constitute the weakened portion 12. In this case, the first part 10a or the second part 10b can be easily manufactured because an entire part of the accommodating portion 10 can be formed as the weakened portion 12, rather than just part of the material constituting the accommodating portion 10.

The shape of the weakened portion 12 is preferably substantially consistent with a cross-sectional shape of the piercing element 130 orthogonal to a longitudinal direction, or larger than this cross-sectional shape. Specifically, the shape of the weakened portion 12 shown in fig. 3(b), seen from the longitudinal direction, is preferably substantially consistent with the cross-sectional shape of the piercing member 132 orthogonal to the longitudinal direction, or larger than this cross-sectional shape. In this case, an opening may be formed by the piercing element 130 only in the weakened portion 12. In other words, it is possible to inhibit an opening from being formed in a part of the accommodating portion 10 other than the weakened portion 12.

The accommodating portion 10 has an elliptical or rounded rectangular external shape, as seen from a direction orthogonal to the longitudinal direction thereof, as shown in fig. 3(a). In this case, the weakened portion 12 is preferably provided on a major axis MA of the elliptical or rounded rectangular shape, as shown in fig. 3. In this case, the weakened portion 12 is positioned on an end face (tip) part of the accommodating portion 10, so the opening can be easily formed in the end face part of the accommodating portion 10, as shown in fig. 3. As a result, the powder accommodated in the accommodating portion 10 can be easily discharged from the opening, and it is therefore possible to inhibit the powder from remaining inside the accommodating portion 10.

Furthermore, in a cross section orthogonal to the direction of insertion (longitudinal direction) of the accommodating portion 10 into the chamber 102, an outer diameter of the piercing element 130 in said cross section is preferably larger than a difference between an inner diameter of the chamber 102 and an outer diameter of the accommodating portion 10. Specifically, this difference corresponds to the total of a width W1 and a width W2 of a gap between the outer circumferential surface of the accommodating portion 10 and the inner face of the enclosure 110 defining the chamber 102, which is shown in fig. 3(a) and 3(b). Furthermore, the outer diameter of the piercing element 130 in the abovementioned cross section corresponds to the diameter of the piercing member 132 in said cross section. In this case, the piercing element 130 can form an opening in the weakened portion 12 even if the position of the accommodating portion 10 is offset inside the chamber 102 in a direction orthogonal to the insertion direction. It should be noted that when the accommodating portion 10 is held by the cartridge 20, as shown in fig. 2, this difference may be taken as the difference between the inner diameter of the chamber 102 and the outer shape of the cartridge 20.

Furthermore, referring to fig. 1 and 2, the powder inhaler 100 preferably comprises the sensor 142, and the control unit 140 which is electrically connected to the sensor 142, as described above. The sensor 142 is configured to detect a pressure applied to the piercing portion 130. Furthermore, the control unit 140 is configured to determine whether or not the piercing element 130 has formed an opening in the weakened portion 12 shown in fig. 3, based on the pressure detected by the sensor 142. In this case, the control unit 140 can determine whether or not an opening could be formed in the weakened portion 12 of the accommodating portion 10, so if an opening has not been formed in the weakened portion 12, a notification to that effect can be provided to the user by emission of vibration, sound, or light, etc. from the powder inhaler 100, for example. The user can take appropriate measures such as replacing the accommodating portion 10 if an opening has not been formed in the weakened portion 12.

The pressure applied to the piercing element 130 is lower when the piercing element 130 forms an opening in the weakened portion 12 than when the piercing element 130 forms and opening in a part of the accommodating portion 10 other than the weakened portion 12. The control unit140 may therefore determine that the piercing element 130 has formed an opening in the weakened portion 12 when the pressure detected by the sensor 142 is equal to or less than a predetermined value. In this case, the user can be notified that the piercing element 130 has formed an opening in the weakened portion 12 by emission of vibration, sound, or light, etc. from the powder inhaler 100, for example. The user can start inhaling having confirmed that an opening has been formed in the weakened portion 12.

### Second Embodiment

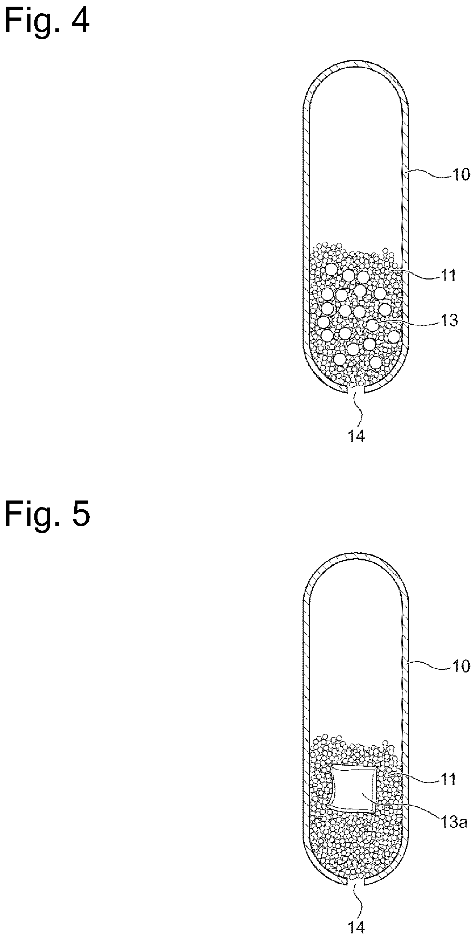

A powder inhalation system 1000 according to a second embodiment will be described next. The powder inhalation system 1000 according to the second embodiment differs from the powder inhalation system 1000 according to the first embodiment in regard to the contents of the accommodating portion 10. That is to say, the powder inhalation system 1000 of the second embodiment comprises an accommodating portion 10 accommodating a powder to be inhaled, and a powder inhaler 100 for inhaling the powder, the powder inhaler 100 having a chamber 102 in which the accommodating portion 10 is disposed. Fig. 4 is a schematic view in side section of the accommodating portion 10 according to the second embodiment. It is conceivable that the powder inside the accommodating portion 10 might absorb water and solidify, which is an unintended situation. For this reason, the accommodating portion 10 according to the second embodiment accommodates a powder 11 and a desiccant 13 which is different from the powder 11, as shown in fig. 4. This makes it possible to inhibit the powder 11 inside the accommodating portion 10 from absorbing moisture and solidifying. An opening 14 formed by the piercing element 130, etc. is shown in the accommodating portion 10 illustrated in fig. 4. The accommodating portion 10 according to the second embodiment may or may not have the weakened portion 12 which was described in the first embodiment.

The desiccant 13 preferably has higher moisture absorption than the powder 11. In this case, the desiccant 13 can efficiently inhibit the powder 11 from absorbing moisture. It should be noted that the moisture absorption may be measured by means of a drying loss method, for example. Specifically, the desiccant 13 preferably comprises at least one selected from the group consisting of: silica gel, sepiolite, calcium oxide, diatomaceous earth, activated charcoal, activated clay, zeolite, white carbon, calcium chloride, magnesium chloride, potassium acetate, disodium phosphate, sodium citrate, and water-absorbent polymers. In this case, a highly water-absorbent material is used as the desiccant 13, so it is possible to further inhibit the powder 11 inside the accommodating portion 10 from absorbing water and solidifying.

As shown in fig. 4, the particle size of the desiccant 13 is preferably greater than the particle size of the powder 11. More specifically, the particle size of the desiccant 13 is preferably larger than the opening 14 formed in the accommodating portion 10. In this case, the desiccant 13 is inhibited from being discharged from the opening 14 formed by the piercing element 130, so the user can be inhibited from inhaling the desiccant 13 by mistake. Furthermore, the hardness of the desiccant 13 is preferably higher than the hardness of the powder 11. In this case, the desiccant 13 collides with the powder 11 inside the accommodating portion 10, thereby making it possible to loosen solidified powder 11. Furthermore, the desiccant 13 itself is inhibited from being broken up and refined, so it is possible to inhibit the refined desiccant 13 from passing through the opening 14 and being supplied to the user. Note that "hardness" as referred to here may be the micro Vickers hardness or Knoop hardness, for example.

Fig. 5 is a schematic view in side section of the accommodating portion 10 according to another example of the second embodiment. As shown in fig. 5, the desiccant 13 may be packaged in an air-permeable member 13a and accommodated in the accommodating portion 10. In this case, the desiccant 13 is packaged, so it is possible to prevent the desiccant 13 from mixing with the powder 11 and to prevent the user from inhaling the desiccant 13. Moreover, a nonwoven fabric or paper, such as plain paper, may be used as the air-permeable member 13a, for example.

### Third Embodiment

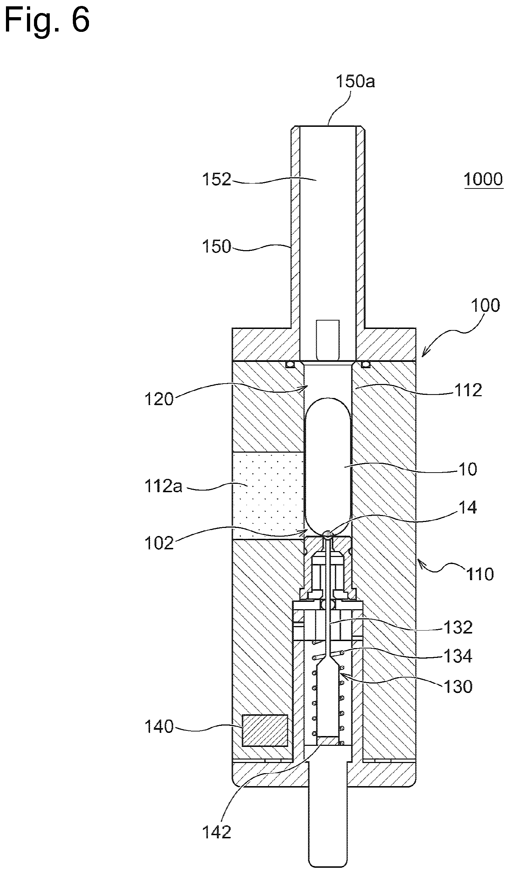

A powder inhalation system 1000 according to a third embodiment will be described next. Fig. 6 is a schematic view in side section showing the powder inhalation system 1000 according to the third embodiment. The powder inhalation system 1000 according to the third embodiment differs from the powder inhalation system 1000 according to the first embodiment in that a wall portion defining a transport line in the powder inhalation system 1000 according to the third embodiment is formed by a transparent or semitransparent material. That is to say, the powder inhalation system 1000 of the third embodiment comprises an accommodating portion 10 and a powder inhaler 100. The powder inhalation system 1000 further comprises: a mouthpiece 150 having an inhalation port 150a; a transport line 120 for transporting the powder to the inhalation port 150a; and a wall portion 112 defining the transport line 120. In the example shown in fig. 6, the mouthpiece 150, the transport line 120, and the wall portion 112 are provided in the powder inhaler 100. The accommodating portion 10 according to the third embodiment may or may not have the weakened portion 12 which was described in the first embodiment.

In the powder inhalation system 1000 such as shown in fig. 6, it could conceivably be difficult to judge whether or not the accommodating portion 10 is spent, and a spent accommodating portion 10 might erroneously be reused. Furthermore, it is also difficult to judge whether or not there is powder remaining in the accommodating portion 10 accommodated in the chamber 102 of the powder inhaler 100. For this reason, in the powder inhalation system 1000 according to the third embodiment, the wall portion 112 defining the transport line 120 is formed by a transparent or semitransparent material which makes the inside of the transport line 120 visible from the outside Specifically, a transparent or semitransparent part 112a is provided on a part of the wall portion 112 of the enclosure 110, as shown in fig. 6. As a result, the powder passing through the transport line 120 is visible from the outside, so it is possible to easily confirm whether or not there is powder remaining inside the accommodating portion 10 or the chamber 102. It is therefore possible to easily judge whether or not the powder inhalation system 1000 is spent.

The wall portion 112 is preferably formed by an antistatic material or coated with an antistatic material. In this case, the wall portion 112 is charged and can thereby inhibit the powder passing through the transport line 120 from adhering to the wall portion 112. This allows the powder to be supplied to the user without waste. It is furthermore possible to inhibit a situation in which the powder passing through the transport line 120 is difficult to see from the transparent or semitransparent part 112a because of adhesion of the powder passing through the transport line 120 to the wall portion 112. An anionic antistatic agent, cationic antistatic agent, amphoteric antistatic agent, or nonionic antistatic agent, etc. may be used as the antistatic material, for example.

As shown in fig. 6, the transparent or semitransparent part 112a is preferably provided in a part of the wall portion 112 surrounding the opening 14 in the accommodating portion 10. In this case, it is possible to easily confirm whether or not the powder is being discharged from the opening 14 in the accommodating portion 10, that is, whether or not there is powder remaining inside the accommodating portion 10.

In the example shown in fig. 6, a part of the wall portion 112 is formed by the transparent or semitransparent part 112a. This is not limiting, and the entirety of the wall portion 112 of the enclosure 110 may equally be formed by the transparent or semitransparent material. In this case, the powder passing through the transport line 120 is visible from the outside over the entirety of the transport line 120, so it is possible to confirm even more easily whether or not there is powder remaining inside the accommodating portion 10 or the chamber 102. It should be noted that the passage 152 in the mouthpiece 150 forms part of the transport line 120, and a part or the entirety of the wall portion defining the passage 152 (corresponding to an example of the transport line) in the mouthpiece 150 may be formed by the transparent or semitransparent material.

The wall portion 112 (transparent or semitransparent part 112a) of the enclosure 110 is preferably formed by at least one selected from the group consisting of an acrylic resin, polycarbonate, polyethylene terephthalate, polyvinyl chloride, and polystyrene. In this case, the wall portion 112 and the transparent or semitransparent part 112a of the enclosure 110 can be formed easily and inexpensively.

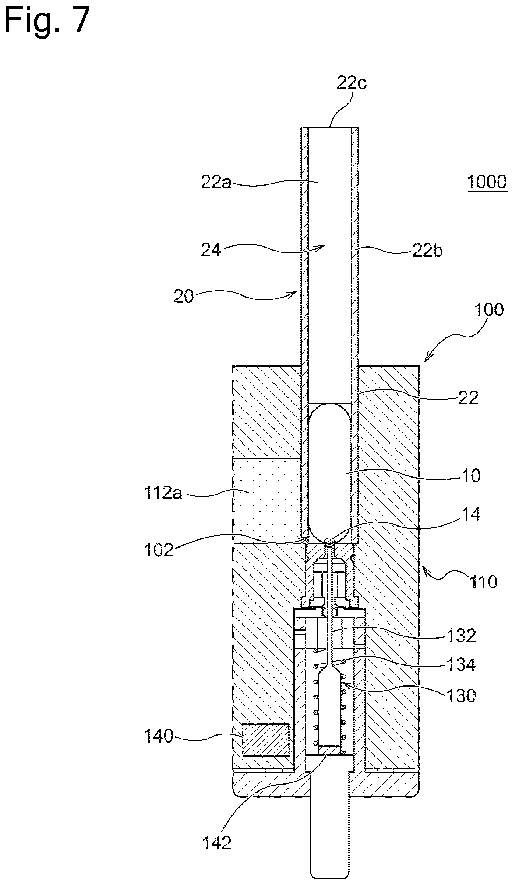

Fig. 7 is a schematic view in side section showing the powder inhalation system 1000 according to another example of the third embodiment. The example shown in fig. 7 differs from the example shown in fig. 6 in that, in the example shown in fig. 7, the accommodating portion 10 is held by means of a cartridge 20, and the powder inhaler 100 does not comprise the mouthpiece 150. Specifically, in the example shown in fig. 7, the cartridge 20 which holds the accommodating portion 10 is detachable from the powder inhaler 100, and a portion of the cartridge 20 extends to outside of the powder inhaler 100 in a state in which the accommodating portion 10 is disposed in the chamber 102, as shown in fig. 7. In this case, the used accommodating portion 10 can be discarded or replaced together with the cartridge 20 comprising the accommodating portion 10. Furthermore, the user can remove the cartridge 20 from the powder inhaler 100 by gripping the part of the cartridge 20 extending to outside of the powder inhaler 100, enabling the user to easily discard or replace the cartridge 20 and the accommodating portion 10.

Furthermore, in the example shown in fig. 7, the cartridge 20 comprises a wall portion 22b formed by a transparent or semitransparent material which makes the inside of the transport line 24 visible from the outside As a result, the powder passing through the transport line 24 in the cartridge 20 is visible from the outside, so it is possible to easily confirm whether or not there is powder remaining inside the accommodating portion 10 or the chamber 102. It is therefore possible to easily judge whether or not the powder inhalation system 1000 is spent. In the example shown in fig. 7, the entirety of the wall portion 22b is formed by the transparent or semitransparent material. This is not limiting, and a part of the wall portion 22b may equally be formed by the transparent or semitransparent material. Specifically, in a state in which the accommodating portion 10 of the cartridge 20 is accommodated in the chamber 102 and inhalation is possible, as shown in fig. 7, for example, only a part of the wall portion 22b overlapping the transparent or semitransparent part 112a of the enclosure 110 in the longitudinal direction may be formed by the transparent or semitransparent material.

The wall portion 22b of the cartridge 20 is preferably formed by at least one selected from the group consisting of glassine paper, parchment paper, paraffin paper, cellophane, polypropylene film, polyvinyl chloride film, and cellulose acetate film. In this case, the wall portion 22b of the cartridge 20 can be formed easily and inexpensively.

The wall portion 22b of the cartridge 20 is preferably formed by an antistatic material or coated with an antistatic material. In this case, the wall portion 22b is charged and can thereby inhibit the powder passing through the transport line 24 from adhering to the wall portion 22b. This allows the powder to be supplied to the user without waste. It is furthermore possible to inhibit a situation in which the powder passing through the transport line 24 is difficult to see from the wall portion 22b because of adhesion of the powder passing through the transport line 24 to the wall portion 22b. An anionic antistatic agent, cationic antistatic agent, amphoteric antistatic agent, or nonionic antistatic agent, etc. may be used as the antistatic material, for example.

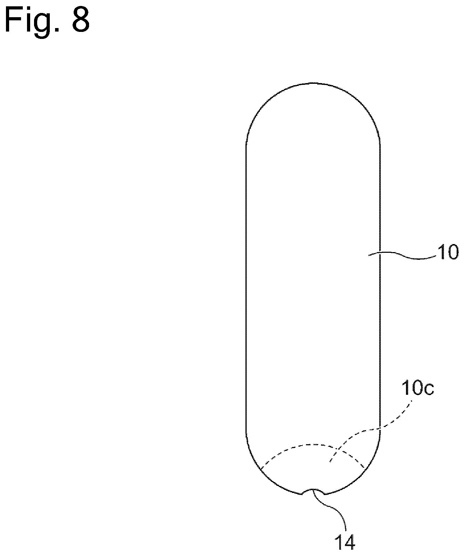

In the example shown in fig. 6 and the example shown in fig. 7, the accommodating portion 10 is preferably formed by a transparent or semitransparent material which makes the inside of the accommodating portion 10 visible from the outside. In this case, the inside of the accommodating portion 10 is visible from the outside, so it is possible to easily confirm whether or not there is powder remaining in the accommodating portion 10. Fig. 8 is a schematic side view showing an example of the accommodating portion 10 in the third embodiment. The part of the accommodating portion 10 in which the opening 14 is formed is preferably formed by the transparent or semitransparent material. Specifically, as shown in fig. 8, the accommodating portion 10 may comprise a transparent or semitransparent part 10c on a portion thereof, and the opening 14 may be formed in this transparent or semitransparent part 10c. In this case, it is possible to visually check from the outside whether or not the powder is being discharged from the opening 14 in the accommodating portion 10, so it is possible to confirm even more easily whether or not there is powder remaining in the accommodating portion 10. This is not limiting, and the entirety of the accommodating portion 10 may be formed by the transparent or semitransparent part 10c.

### Fourth Embodiment

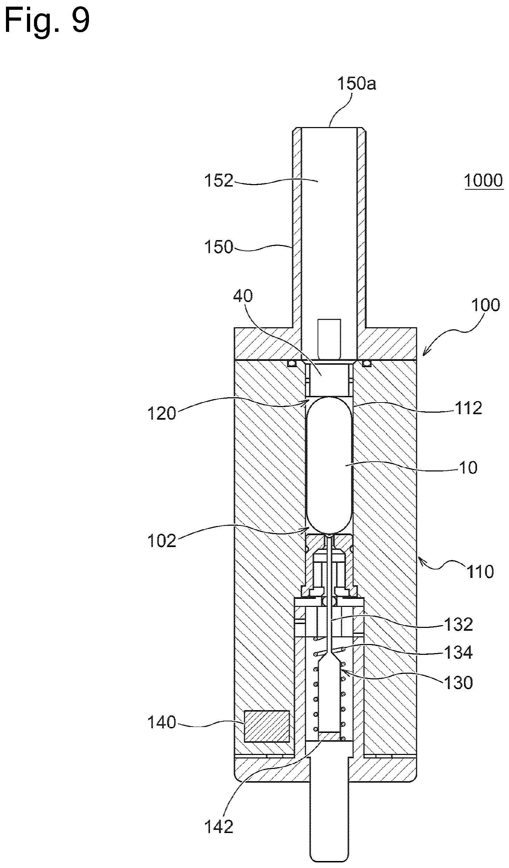

A powder inhalation system 1000 according to a fourth embodiment will be described next. Fig. 9 is a schematic view in side section showing the powder inhalation system 1000 according to the fourth embodiment. The powder inhalation system 1000 according to the fourth embodiment differs from the powder inhalation system 1000 according to the first embodiment in that the powder inhalation system 1000 according to the fourth embodiment comprises an obstructing portion for obstructing part of a flow of powder when the user inhales. That is to say, the powder inhalation system 1000 of the fourth embodiment comprises an accommodating portion 10 and a powder inhaler 100. The powder inhalation system 1000 further comprises a mouthpiece 150 and a transport line 120. The powder inhaler 100 comprises a chamber 102 and a piercing element 130. In the example shown in fig. 9, the mouthpiece 150 and the transport line 120 are provided in the powder inhaler 100. The accommodating portion 10 according to the fourth embodiment may or may not have the weakened portion 12 which was described in the first embodiment.

In the powder inhalation system 1000 such as shown in fig. 9, there is a risk of the powder inside the accommodating portion 10 absorbing water and solidifying, and of the solidified powder being inhaled by the user. The powder inhalation system 1000 according to the fourth embodiment therefore comprises an obstructing portion for obstructing part of the flow of powder when the user inhales. As a result, even if the powder inside the accommodating portion 10 absorbs water to form clumps, the obstructing portion can inhibit such clumps from reaching the user's mouth. Specifically, in the example shown in fig. 9, a filter 40 constituting an example of an obstructing portion may be disposed in the transport line 120 in the enclosure 110. The filter 40 may be provided in the passage 152 (corresponding to an example of the transport line) inside the mouthpiece 150.

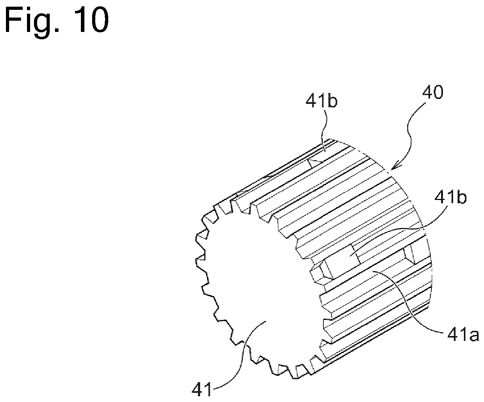

Fig. 10 is an oblique view of the filter 40. As shown in fig. 10, the filter 40 comprises a columnar body 41 having a plurality of grooves 41a on an outer circumferential surface thereof. The plurality of grooves 41a may extend toward the inhalation port 150a of the mouthpiece 150, and at least one of the plurality of grooves 41a may have a dam portion 41b provided within said groove. In this case, the powder reaches the inhalation port 150a by passing through the plurality of grooves 41a on the outer circumferential surface, but clumps of powder can be inhibited from reaching the inhalation port 150a by means of the dam portion 41b. This makes it possible to inhibit clumps of powder from reaching the user's mouth. The filter 40 may be formed by paper, a nonwoven fabric, or cellulose acetate, etc., for example.

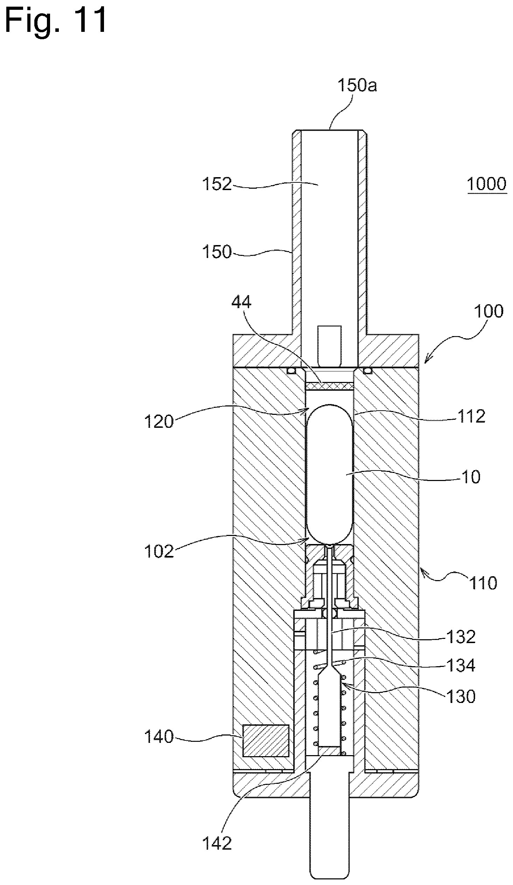

Fig. 11 is a schematic view in side section showing another example of the powder inhalation system 1000 according to the fourth embodiment. In the example shown in fig. 11, a mesh filter 44 constituting an example of an obstructing portion may be disposed in the transport line 120 in the enclosure 110. In this case, the powder passes through the mesh filter 44 and reaches the inhalation port 150a, but clumps of powder can be inhibited from reaching the inhalation port 150a by the mesh filter 44. This makes it possible to inhibit clumps of powder from reaching the user's mouth. The mesh filter 44 may be provided in the passage 152 (corresponding to an example of the transport line) inside the mouthpiece 150. Note that the mesh filter 44 may be, for example, a metal mesh filter, a fiber layer filter, or a particle-packed layer filter, etc. The opening size, wire diameter, fiber diameter, particle size, packing ratio, or packing length, etc. of the mesh filter 44 may be modified so that the efficiency of trapping clumps of powder is optimally designed.

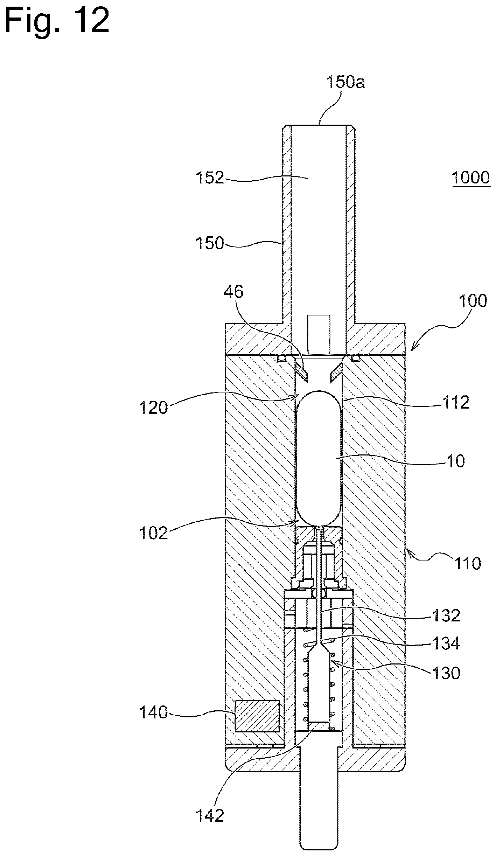

Fig. 12 is a schematic view in side section showing another example of the powder inhalation system 1000 according to the fourth embodiment. In the example shown in fig. 12, a collision plate 46 constituting an example of an obstructing portion may be disposed in the transport line 120 in the enclosure 110. The collision plate 46 is provided on the inner face of the wall portion 112 defining the transport line 120, and forms an opening in the center of the transport line 120. In this case, the powder passes through the open central part of the transport line 120 to reach the inhalation port 150a, but clumps of powder can be inhibited from reaching the inhalation port 150a by the collision plate 46. This makes it possible to inhibit clumps of powder from reaching the user's mouth. Note that the collision plate 46 may be provided in the passage 152 (corresponding to an example of the transport line) inside the mouthpiece 150. In the example shown in fig. 12, the collision plate 46 is inclined toward the accommodating portion 10. In this case, the inclined collision plate 46 can efficiently inhibit clumps of powder from reaching the inhalation port 22c. The collision plate 46 may extend in a direction orthogonal to the longitudinal direction, without being inclined. Furthermore, the collision plate 46 may be provided on a part of the inner face of the wall portion 112, but is preferably provided over the entire circumference of the inner face of the wall portion 112.

An obstructing portion is provided in the powder inhaler 100 in the powder inhalation system 1000 illustrated in fig. 9-12, as described above. In this case, there is no need to provide an obstructing portion in the cartridge 20, so the obstructing portion can be used repeatedly. On the other hand, an obstructing portion may be provided in the cartridge 20.

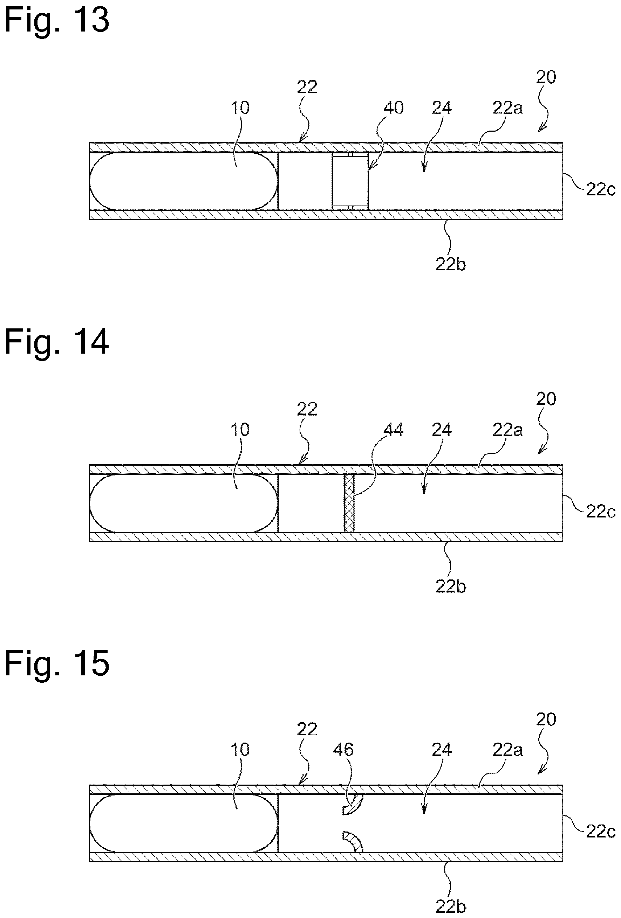

Fig. 13-15 are schematic views in side section of examples of the cartridge 20 according to the fourth embodiment. The cartridges 20 shown in fig. 13-15 may be used in the powder inhaler 100 shown in fig. 2. In the example shown in fig. 13, the cartridge 20 which holds the accommodating portion 10 comprises the filter 40 disposed in the transport line 24. In this case, the filter 40 constituting an obstructing portion is provided in the cartridge 20 which is replaced as appropriate, so there is no need to clean the filter 40 even if powder adheres thereto. The filter 40 may be the same as the filter 40 shown in fig. 9 and 10.

In the example shown in fig. 14, the cartridge 20 which holds the accommodating portion 10 comprises the mesh filter 44 disposed in the transport line 24. In this case, the mesh filter 44 constituting an obstructing portion is provided in the cartridge 20 which is replaced as appropriate, so there is no need to clean the mesh filter 44 even if powder adheres thereto. The mesh filter 44 may be the same as the mesh filter 44 shown in fig. 11.

In the example shown in fig. 15, the cartridge 20 which holds the accommodating portion 10 comprises the collision plate 46 disposed in the transport line 24. In this case, the collision plate 46 constituting an obstructing portion is provided in the cartridge 20 which is replaced as appropriate, so there is no need to clean the collision plate 46 even if powder adheres thereto. In the example shown in fig. 15, the collision plate 46 is curved toward the accommodating portion 10. In this case, the curved collision plate 46 can efficiently inhibit clumps of powder from reaching the inhalation port 22c. The collision plate 46 may extend in a direction orthogonal to the longitudinal direction, without being curved. Furthermore, the collision plate 46 may be provided on a part of the inner face of the wall portion 22b of the tubular member 22, but is preferably provided over the entire circumference of the inner face of the wall portion 22b.

The obstructing portion shown in fig. 9-15 is preferably formed by an antistatic material or coated with an antistatic material. In this case, the obstructing portion is charged and can thereby inhibit the powder passing through the transport line 24 or the transport line 120 from adhering to the obstructing portion. This allows the powder to be supplied to the user without waste. An anionic antistatic agent, cationic antistatic agent, amphoteric antistatic agent, or nonionic antistatic agent, etc. may be used as the antistatic material, for example.

The obstructing portion shown in fig. 9-15 is preferably formed by a non-water-absorbent material or coated with a non-water-absorbent material. In this case, it is possible to inhibit the powder solidifying as a result of the powder absorbing water when the powder collides with the obstructing portion. Furthermore, it is possible to inhibit the powder from adhering to the obstructing portion when the powder collides with the obstructing portion. This makes it possible to supply the powder to the user without waste, while inhibiting clumps of powder from reaching the user's mouth. It should be noted that polyethylene or polypropylene, etc. may be used as the non-water-absorbent material, for example.

Embodiments of the present invention were described above, but the present invention is not limited to those embodiments, and various modifications are possible within the scope of the technical concept disclosed in the claims, specification and drawings. Moreover, any shape or material not directly stated in the specification or drawings is also within the scope of the technical concept of the invention of this application, provided that it exhibits the action and effect of the invention of this application. For example, features of the embodiments above may be combined with each other.

Several aspects disclosed in the present specification are provided below.
1-1 A powder inhalation system comprising an accommodating portion accommodating a powder to be inhaled; and a powder inhaler for inhaling the powder, wherein
   the powder inhaler has a chamber in which the accommodating portion is disposed, and
   the accommodating portion has a weakened portion with a lower breaking strength than other parts of the accommodating portion.
1-2 The powder inhalation system as disclosed in 1-1,
   comprising a piercing element configured to form an opening in the accommodating portion disposed in the chamber,
   the piercing element being configured to form the opening in the weakened portion.
1-3 The powder inhalation system as disclosed in 1-2, wherein
   the shape of the weakened portion is substantially consistent with a cross-sectional shape of the piercing element orthogonal to a longitudinal direction, or larger than this cross-sectional shape.
1-4 The powder inhalation system as disclosed in 1-2 or 1-3, wherein
   the accommodating portion has an elliptical or rounded rectangular external shape, as seen from a direction orthogonal to the longitudinal direction thereof, and
   the weakened portion is provided on a major axis of the elliptical or rounded rectangular shape.
1-5 The powder inhalation system as disclosed in any of 1-2 to 1-4, wherein
   in a cross section orthogonal to a direction of insertion of the accommodating portion into the chamber, an outer diameter of the piercing element in said cross section is larger than a difference between an inner diameter of the chamber and an outer diameter of the accommodating portion.
1-6 The powder inhalation system as disclosed in any of 1-2 to 1-5, wherein
   the powder inhaler comprises:
   a sensor for detecting a pressure applied to the piercing element; and
   a control unit for determining whether or not the piercing portion has formed an opening in the weakened portion, based on the pressure detected by the sensor.
1-7 The powder inhalation system as disclosed in 1-6, wherein
   the control unit determines that the piercing element has formed an opening in the weakened portion when the pressure detected by the sensor is equal to or less than a predetermined value.
1-8 The powder inhalation system as disclosed in any of 1-1 to 1-7, wherein
   the weakened portion is formed by a different material from that of other parts of the accommodating portion.
1-9 The powder inhalation system as disclosed in any of 1-1 to 1-8, wherein
   the thickness of the weakened portion is smaller than the thickness of other parts of the accommodating portion.
1-10 The powder inhalation system as disclosed in any of 1-1 to 1-9, wherein
   the accommodating portion includes a first part and a second part configured to be detachable from the first part, and
   the weakened portion is provided on either one of the first part and the second part.
1-11 The powder inhalation system as disclosed in 1-10, wherein
   the entirety of either one of the first part and the second part constitutes the weakened portion.
1-12 The powder inhalation system as disclosed in any of 1-1 to 1-11, wherein
   the accommodating portion is detachably received in the chamber of the powder inhaler.
1-13 The powder inhalation system as disclosed in any of 1-1 to 1-11, wherein
   the accommodating portion is integrally formed with the powder inhaler.
1-14 The powder inhalation system as disclosed in any of 1-1 to 1-13, comprising:
   a mouthpiece having an inhalation port; and
   a transport line for transporting the powder in the accommodating portion toward the inhalation port.
1-15 The powder inhalation system as disclosed in any of 1-1 to 1-12,
   comprising a cartridge which holds the accommodating portion and is detachable from the powder inhaler,
   wherein a portion of the cartridge extends to outside of the powder inhaler in a state in which the accommodating portion is disposed in the chamber.
2-1 A powder inhalation system comprising an accommodating portion accommodating a powder to be inhaled; and a powder inhaler for inhaling the powder, wherein
   the powder inhaler has a chamber in which the accommodating portion is disposed, and
   the accommodating portion accommodates a desiccant different from the powder.
2-2 The powder inhalation system as disclosed in 2-1, wherein
   the desiccant has higher moisture absorption than the powder.
2-3 The powder inhalation system as disclosed in 2-1 or 2-2,
   comprising a piercing element configured to form an opening in the accommodating portion disposed in the chamber.
2-4 The powder inhalation system as disclosed in 2-3, wherein
   the particle size of the desiccant is larger than the opening formed in the accommodating portion.
2-5 The powder inhalation system as disclosed in any of 2-1 to 2-4, wherein
   the hardness of the desiccant is higher than the hardness of the powder.
2-6 The powder inhalation system as disclosed in any of 2-1 to 2-5, wherein
   the desiccant is packaged in an air-permeable member and accommodated in the accommodating portion.
2-7 The powder inhalation system as disclosed in any of 2-1 to 2-6, wherein
   the desiccant comprises at least one selected from the group consisting of: silica gel, sepiolite, calcium oxide, diatomaceous earth, activated charcoal, activated clay, zeolite, white carbon, calcium chloride, magnesium chloride, potassium acetate, disodium phosphate, sodium citrate, and water-absorbent polymers.
2-8 The powder inhalation system as disclosed in any of 2-1 to 2-7, wherein
   the accommodating portion is detachably received in the chamber of the powder inhaler.
2-9 The powder inhalation system as disclosed in any of 2-1 to 2-8, wherein
   the accommodating portion is integrally formed with the powder inhaler.
2-10 The powder inhalation system as disclosed in any of 2-1 to 2-9, comprising:
   a mouthpiece having an inhalation port; and
   a transport line for transporting the powder in the accommodating portion toward the inhalation port.
2-11 The powder inhalation system as disclosed in any of 2-1 to 2-8,
   comprising a cartridge which holds the accommodating portion and is detachable from the powder inhaler,
   wherein a portion of the cartridge extends to outside of the powder inhaler in a state in which the accommodating portion is disposed in the chamber.
3-1 A powder inhalation system comprising: an accommodating portion accommodating a powder to be inhaled; a mouthpiece having an inhalation port; a transport line for transporting the powder inside the accommodating portion from the accommodating portion to the inhalation port; a wall portion defining the transport line; and a powder inhaler for inhaling the powder, wherein the powder inhaler has a chamber in which the accommodating portion is disposed, and
   a piercing element configured to form an opening in the accommodating portion disposed in the chamber, and
   the wall portion defining the transport line is formed by a transparent or semitransparent material which makes the inside of the transport line visible from the outside
3-2 The powder inhalation system as disclosed in 3-1,
   comprising a cartridge for holding the accommodating portion, at least a portion of the cartridge extending to outside of the powder inhaler,
   wherein the cartridge is detachable from the powder inhaler and comprises the mouthpiece, the transport line, and the wall portion.
3-3 The powder inhalation system as disclosed in 3-2, wherein
   the wall portion of the cartridge is formed by at least one selected from the group consisting of glassine paper, parchment paper, paraffin paper, cellophane, polypropylene film, polyvinyl chloride film, and cellulose acetate film.
3-4 The powder inhalation system as disclosed in 3-2 or 3-3, wherein
   the wall portion of the cartridge is formed by an antistatic material or coated with an antistatic material.
3-5 The powder inhalation system as disclosed in 3-1, wherein
   the accommodating portion is integrally formed with the powder inhaler.
3-6 The powder inhalation system as disclosed in any of 3-1 to 3-4, wherein
   the accommodating portion is detachably received in the chamber of the powder inhaler.
3-7 The powder inhalation system as disclosed in any of 3-1 to 3-6, wherein
   the powder inhaler comprises the transport line and the wall portion.
3-8 The powder inhalation system as disclosed in 3-7, wherein
   the entirety of the wall portion of the powder inhaler is formed by the transparent or semitransparent material.
3-9 The powder inhalation system as disclosed in 3-7 or 3-8, wherein
   the wall portion of the powder inhaler is formed by at least one selected from the group consisting of an acrylic resin, polycarbonate, polyethylene terephthalate, polyvinyl chloride, and polystyrene.
3-10 The powder inhalation system as disclosed in any of 3-1 to 3-9, wherein
   the accommodating portion is formed by a transparent or semitransparent material which makes the inside of the accommodating portion visible from the outside.
3-11 The powder inhalation system as disclosed in 3-10, wherein
   the part of the accommodating portion in which the opening is formed is formed by the transparent or semitransparent material.
4-1 A powder inhalation system comprising an accommodating portion accommodating a powder to be inhaled; and a powder inhaler for inhaling the powder, wherein the powder inhalation system comprises:
   a mouthpiece having an inhalation port; and
   a transport line for transporting the powder in the accommodating portion toward the inhalation port;
   the powder inhaler comprises:
      a chamber in which the accommodating portion is disposed, and
      a piercing element configured to form an opening in the accommodating portion disposed in the chamber, and
   the flavor inhaler system has an obstructing portion which is disposed in the transport line and obstructs part of a flow of powder when the user inhales.
4-2 The powder inhalation system as disclosed in 4-1, wherein
   the obstructing portion comprises a columnar body having a plurality of grooves on an outer circumferential surface thereof,
   the plurality of grooves extend toward the inhalation port, and
   at least one of the plurality of grooves has a dam portion provided within said groove.
4-3 The powder inhalation system as disclosed in 4-1, wherein
   the obstructing portion comprises a mesh filter.
4-4 The powder inhalation system as disclosed in 4-1, wherein
   the obstructing portion comprises a collision plate which is provided on an inner face of the wall portion defining the transport line, and forms an opening in the center of the transport line.
4-5 The powder inhalation system as disclosed in 4-4, wherein
   the collision plate is curved or inclined toward the accommodating portion.
4-6 The powder inhalation system as disclosed in any of 4-1 to 4-5, wherein
   the obstructing portion is formed by an antistatic material or coated with an antistatic material.
4-7 The powder inhalation system as disclosed in any of 4-1 to 4-5, wherein
   the obstructing portion is formed by a non-water-absorbent material or coated with a non-water-absorbent material.
4-8 The powder inhalation system as disclosed in any of 4-1 to 4-7, wherein
   the accommodating portion is detachably received in the chamber of the powder inhaler.
4-9 The powder inhalation system as disclosed in any of 4-1 to 4-8, wherein
   the accommodating portion is integrally formed with the powder inhaler.
4-10 The powder inhalation system as disclosed in any of 4-1 to 4-8,
   having a cartridge which holds the accommodating portion and comprises the transport line,
   wherein the obstructing portion is provided in the cartridge.
4-11 The powder inhalation system as disclosed in 4-10,
   wherein a portion of the cartridge extends to outside of the powder inhaler in a state in which the accommodating portion is disposed in the chamber.
4-12 The powder inhalation system as disclosed in any of 4-1 to 4-9, wherein
   the powder inhaler comprises the transport line, and
   the obstructing portion is provided in the powder inhaler.

### REFERENCE SIGNS LIST

10 Accommodating portion
11 Powder
13 Dessicant
13a Air-permeable member
14 Opening
20 Cartridge
22a Mouthpiece
22c Inhalation port
24 Transport line
100 Powder inhaler
102 Chamber
120 Transport line
130 Piercing element
150 Mouthpiece
150a Inhalation port
1000 Powder inhalation system

## Claims

1. A powder inhalation system comprising an accommodating portion accommodating a powder to be inhaled; and a powder inhaler for inhaling the powder, wherein
the powder inhaler has a chamber in which the accommodating portion is disposed, and
the accommodating portion accommodates a desiccant different from the powder.

2. The powder inhalation system as claimed in claim 1, wherein
the desiccant has higher moisture absorption than the powder.

3. The powder inhalation system as claimed in claim 1 or 2,
comprising a piercing element configured to form an opening in the accommodating portion disposed in the chamber.

4. The powder inhalation system as claimed in claim 3, wherein
the particle size of the desiccant is larger than the opening formed in the accommodating portion.

5. The powder inhalation system as claimed in any one of claims 1 to 4, wherein
the hardness of the desiccant is higher than the hardness of the powder.

6. The powder inhalation system as claimed in any one of claims 1 to 5, wherein
the desiccant is packaged in an air-permeable member and accommodated in the accommodating portion.

7. The powder inhalation system as claimed in any one of claims 1 to 6, wherein
the desiccant comprises at least one selected from the group consisting of: silica gel, sepiolite, calcium oxide, diatomaceous earth, activated charcoal, activated clay, zeolite, white carbon, calcium chloride, magnesium chloride, potassium acetate, disodium phosphate, sodium citrate, and water-absorbent polymers.

8. The powder inhalation system as claimed in any one of claims 1 to 7, wherein
the accommodating portion is detachably received in the chamber of the powder inhaler.

9. The powder inhalation system as claimed in any one of claims 1 to 8, wherein
the accommodating portion is integrally formed with the powder inhaler.

10. The powder inhalation system as claimed in any one of claims 1 to 9, comprising:
a mouthpiece having an inhalation port; and
a transport line for transporting the powder in the accommodating portion toward the inhalation port.

11. The powder inhalation system as claimed in any one of claims 1 to 8,
comprising a cartridge which holds the accommodating portion and is detachable from the powder inhaler,
wherein a portion of the cartridge extends to outside of the powder inhaler in a state in which the accommodating portion is disposed in the chamber.
